# EUROPEAN PATENT APPLICATION

(11) **EP 1 123 919 A1**
(43) Date of publication of application: **16.08.2001**
(21) Application number: 99949341.4
(22) Date of filing: 20.10.1999
(51) Int. Cl.: C07C 231/12, C07C 237/04, C07C 269/06, C07C 271/22, C07C 311/19

(54) **PROCESS FOR PRODUCING PEPTIDYL ALDEHYDES**

(30) Priority: 23.10.1998 JP 30280798
(71) Applicant: Senju Pharmaceutical Co., Ltd., Osaka-shi, Osaka 541-0046 (JP)
(72) Inventor: INOUE, Jun, 603, Rui-Shatore Suma Myodani, Kobe-shi, Hyogo 654-0101 (JP); SAKAI, Yusuke, Kobe-shi, Hyogo 651-2111 (JP)
(74) Representative: von Kreisler, Alek, Dipl.-Chem.
(86) International application number: JP9905810
(87) International publication number: WO0024704

(57) **Abstract**

The present invention provides a method of producing a peptidyl aldehyde derivative of the formula (II) 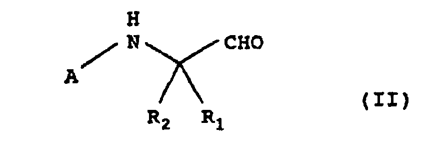 wherein A is acyl derived from amino acid, and R₁ and R₂ are different and one is hydrogen atom and the other is optionally substituted lower alkyl, which includes oxidizing a peptidyl alcohol derivative of the formula (I) 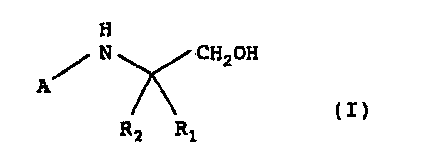 wherein each symbol is as defined above, with DMSO in the presence of diisopropylethylamine. According to the present invention, formation of a stereoisomer can be prevented or suppressed.

## Description

### Technical Field

The present invention relates to a method of producing peptidylaldehyde derivatives, which is free of formation of a stereoisomer or which markedly suppresses formation of a stereoisomer.

### Background Art

Peptidylaldehyde derivatives have various physiological activities and have been elucidated to be useful as inhibitors of cysteine protease, which is one of the proteases, and the like.

Ever since leupeptin was isolated from a culture medium of bacteria belonging to the genus Streptomyces, various peptide derivatives, which are leupeptin analogs, have been synthesized (US5081284, US5510531, EP520336 and the like). Because peptidylaldehyde derivatives have a higher cysteine protease inhibitory activity than do other non-peptide derivatives (e.g., US5554767, US5843992), peptide non-aldehyde derivative (e.g., EP802909) and the like, all having a cysteine protease activity, synthesis of peptide derivatives, particularly peptidylaldehyde derivatives, has been actively tried.

When peptidylaldehyde derivatives are to be synthesized, aldehyde is synthesized by oxidizing the corresponding peptidyl alcohol in the final step, because aldehyde is chemically instable. Heretofore, Swern Oxidation in the presence of triethylamine, Parikh-Doering Oxidation and the like have been used for oxidation. However, the resulting product, a peptidylaldehyde derivative, undergoes epimerization of α-carbon of aldehyde in the reaction mixture (EP572547), which necessitates troublesome purification (separation of diastereomer) in the final stage and results in a low yield.

To eliminate separation of a diastereomer, a different method for synthesizing peptidylaldehyde derivatives is known, which includes conversion of carboxylic group of amino acid into a compound having N-alkyloxy-N-alkylamide, followed by reduction to aldehyde (EP731107). This method, too, increases the number of steps and requires an anhydrous reaction system for reduction, which is not necessarily convenient.

Further, a method including treating peptidylacetal with an acid to convert same to aldehyde is also known. However, this method also increases the number of steps and may form a diketopiperazine derivative during the treatment with an acid. As such, this method is not the most appropriate method.

### Disclosure of the Invention

It is therefore an object of the present invention to develop a convenient production method of peptidylaldehyde derivative for the production of a highly pure peptidylaldehyde derivative in a high yield, which method being free of or markedly suppresses the formation of a stereoisomer.

The present inventors have conducted intensive studies in an attempt to solve the above-mentioned problems and surprisingly found that the formation of a stereoisomer can be markedly suppressed by the use of diisopropylethylamine as an organic base in a solvent during oxidation with activated dimethyl sulfoxide (DMSO), particularly that by Parikh-Doering Oxidation, for the production of aldehyde derivative, which resulted in the completion of the present invention.

Accordingly, the present invention relates to a method of producing a peptidylaldehyde derivative of the following formula (II) 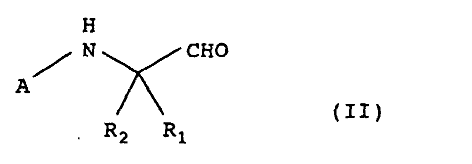 wherein A is acyl derived from amino acid, and R₁ and R₂ are different and one is hydrogen atom and the other is optionally substituted lower alkyl [hereinafter to be also referred to as aldehyde (II)], which comprises oxidizing a peptidyl alcohol derivative of the formula (I) 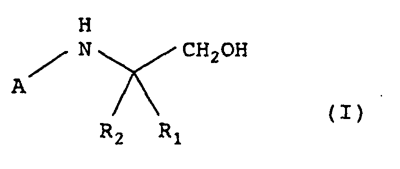 wherein A is acyl derived from amino acid, and R₁ and R₂ are different and one is hydrogen atom and the other is optionally substituted lower alkyl [hereinafter to be also referred to as alcohol (I)] with activated DMSO in the presence of diisopropylethylamine. According to the production method of the present invention, a stereoisomer is not formed due to the asymmetric carbon, to which R₁ and R₂ are bonded in the formula (II), or formation of a stereoisomer is markedly suppressed.

### Detailed Description Of The Invention

In the formulas (I) and (II), "lower alkyl" means linear or branched alkyl having 1 to 6 carbon atoms, and "alkoxy" means linear or branched alkoxy having 1 to 6 carbon atoms, unless particularly specified.

In the formulas (I) and (II), amino acid, from which acyl as designated by A is derived, means natural amino acid, non-natural amino acid or an amino acid derivative.

Examples of the natural amino acid include glycine, alanine, serine, cysteine, homocysteine, cystine, threonine, valine, methionine, leucine, isoleucine, phenylalanine, tyrosine, proline, hydroxyproline, tryptophane, aspartic acid, glutamic acid, arginine, lysine, ornithine, histidine, asparagine, glutamine and the like.

The non-natural amino acid means those wherein the side chain of the above-mentioned natural amino acid is chemically modified with, for example, linear or branched alkyl having 1 to 12 carbon atoms, cyclic alkyl having 3 to 8 carbon atoms, alkoxy, aryl (phenyl, benzyl, naphthyl, anththryl, benzhydryl and the like), heteromonocyclic residue (thienyl, thiazolyl, imidazolyl, pyridyl and the like), condensed heterocyclic residue (indolyl, quinolyl, benzothiophenyl, benzofuranyl and the like), a generally used side chain protecting group (benzyloxycarbonyl, carbamyl, benzyl and the like), halogen atom (fluorine atom, chlorine atom, bromine atom, iodine atom and the like), oxygen atom, nitrogen atom and the like, and those wherein an atom, such as hydrogen atom and the like, has been released from the above-mentioned natural amino acid. Specific examples of the non-natural amino acid include norleucine, phenylglycine, cyclohexylglycine, 4-thiazolylalanine, dehydroalanine, 3-(3-pyridyl)alanine, β-(2-thienyl)serine, S-methylcysteine, S-benzylhomocysteine, O-benzylthreonine, α-methylvaline, methionine sulfoxide, α-methylmethionine, cycloleucine, alloisoleucine, 6-diazo-5-oxonorleucine, biphenylalanine, 3,5-dibromotyrosine, 5-tert-butylproline, 3,4-dehydroproline, 4-hydroxy-3,3-dimethylproline, 4-methyltryptophane, 5-methoxytryptophane, threo-β-methylaspartic acid, 4-fluoroglutamic acid, δ-benzyloxycarbonylornithine, N-deltacarbanylornithine, 3-methylhistidine, 1-methylhistidine and the like.

The above-mentioned natural amino acid and non-natural amino acid may be a D compound or L compound.

The amino acid derivative means those wherein the N terminal hydrogen atom of the above-mentioned natural amino acid and non-natural amino acid is substituted by a substituent and peptide.

Examples of the substituent include linear or branched alkyl having 1 to 12 carbon atoms, cyclic alkyl having 3 to 8 carbon atoms, alkoxy, aryl (phenyl, benzyl, naphthyl, anthryl, benzhydryl and the like), heteromonocyclic residue (thienyl, thiazolyl, imidazolyl, pyridyl and the like), condensed heterocyclic residue (indolyl, quinolyl, benzothiophenyl, benzofuranyl and the like), generally used N-protecting group, alkylsulfonyl, arylsulfonyl, acyl and the like.

Examples of the generally used N-protecting group include benzyloxycarbonyl, tert-butoxycarbonyl and 9-fluorenylmethoxycarbonyl. The alkyl moiety of alkyl sulfonyl is lower alkyl. The aryl moiety of arylsulfonyl may be, for example, phenyl, naphthyl and pentaphenyl optionally substituted by lower alkyl, alkoxy, halogen atom (chlorine atom, fluorine atom, bromine atom and the like), hydroxy, nitro, amino, trifluoromethyl and the like, indenyl, azulenyl and the like. Examples of acyl include acetyl, benzoyl and the like.

As used in this specification, the peptide encompassed in the amino acid derivative means peptide having 2 to 4 above-mentioned natural amino acid and/or non-natural amino acid as necessary. The amino acid derivative also encompasses such peptide having an N terminal hydrogen atom substituted by the above-mentioned substituent, as does the aforementioned amino acid derivative.

In the above-mentioned formulas (I) and (II), the substituent of the optionally substituted lower alkyl at R₁ or R₂ includes, for example, aryl and aromatic heterocyclic residue. Examples of the aryl include phenyl, 1-naphthyl, 2-naphthyl and the like. As the aromatic heterocyclic residue, heteromonocyclic residue and condensed heterocyclic residue having oxygen, nitrogen and/or sulfur atom(s) are exemplified. The heteromonocyclic residue may be, for example, pyrrolyl, furyl, thienyl, oxazolyl, thiazolyl, imidazolyl, pyrazolyl, pyridyl and the like, and the condensed heterocyclic residue may be, for example, indolyl, quinolyl, benzothiophenyl, benzofuranyl, indazolyl, quinazolynyl, phtharazinyl, quinoxalinyl and the like.

Specific examples of the optionally substituted lower alkyl at R₁ or R₂ include isobutyl, benzyl, cyclohexylmethyl, indol-3-ylmethyl and the like.

As used in this specification, the stereoisomer includes optical isomer and diastereomer.

The production method of the peptidylaldehyde derivative of the present invention includes dissolving a peptidyl alcohol derivative of the formula (I) 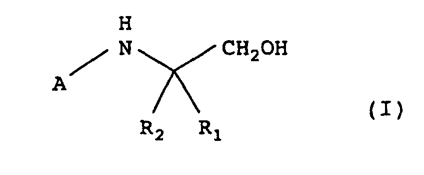 wherein A is acyl derived from amino acid, and R₁ and R₂ are different and one is hydrogen atom and the other is optionally substituted lower alkyl, in DMSO alone or a mixed solvent of DMSO and a solvent (e.g., tetrahydrofuran, dichloromethane, chloroform, ethyl acetate, benzene, ether and the like) that does not inhibit the oxidation, and adding diisopropylethylamine in an amount of generally 1 to 10-fold moles, preferably 3 to 6-fold moles, per 1 mole of the above-mentioned alcohol (I).

The amount used of DMSO in the above is 1 - 100 ml, preferably 5 - 20 ml, per 1 g of alcohol (I).

According to the method of the present invention, the use of DMSO is essential, but it is also possible to use a mixed solvent of DMSO and a solvent that does not inhibit the above-mentioned oxidation. The amount mixed of the solvent that does not inhibit the above-mentioned oxidation is not subject to any particular limitation as long as the peptidyl alcohol derivative of the formula (I) is dissolved in the mixed solvent without precipitation. For example, a 100-fold volume, preferably 5-fold volume, can be used per 1 volume of DMSO upon mixing with DMSO. In addition, two or more kinds of solvents, that do not inhibit the above-mentioned oxidation reaction, can be used upon mixing, in which case each solvent is added in the above-mentioned volume relative to the volume of DMSO.

The total amount of the solvent used can be any as long as it permits easy stirring of the reaction mixture, such as 1 - 1000 ml, preferably 1 - 300 ml, more preferably 5 - 50 ml, per 1 g of alcohol (I).

Diisopropylethylamine is added at a temperature of from -20°C to 50°C, preferably from 0°C to 30°C.

Then, an activator is added to activate DMSO for oxidation to give a peptidylaldehyde derivative of the formula (II) 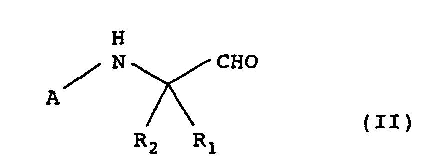 wherein A, R₁ and R₂ are as defined above. This product is free of formation of a stereoisomer due to an asymmetric carbon, to which R₁ and R₂ are bonded, or shows extremely suppressed formation of a stereoisomer. When compared with alcohol (I), the objective peptidylaldehyde derivative wherein the configuration of the asymmetric carbon, to which R₁ and R₂ are bonded, is not inverted, can be produced with a high purity.

Examples of the activator of the above-mentioned DMSO, which can be used advantageously, include a sulfur trioxide-pyridine complex, oxalyl chloride, dicyclohexylcarbodiimide, acetic anhydride and the like, wherein particularly preferable is a sulfur trioxide-pyridine complex.

The activator is used in an amount of 1 to 20-fold moles, preferably 2 to 10-fold moles, relative to 1 mole of alcohol (I). For example, in the case of a sulfur trioxide-pyridine complex, it is used in an amount of 3 to 6-fold moles per 1 mole of alcohol (I).

The activator is preferably dissolved or suspended in 1 - 20 ml of a solvent (e.g., DMSO, dichloromethane, benzene, ether etc.) per 1 g of the activator and added to the reaction mixture with stirring. The temperature, at which the activator is added, is from -20°C to 50°C, preferably from 0°C to 30°C.

The reaction temperature after addition of the activator is not subject to any particular limitation, but it is generally under cooling, at room temperature or under heating.

The reaction time is 10 min - 24 hr, preferably 30 min - 5 hr. The termination of the reaction can be confirmed by the analysis method generally used, such as thin-layer chromatography, high performance liquid chromatography (HPLC) and the like.

After the completion of the reaction, the peptidylaldehyde derivative can be isolated and purified by a purification method generally used, such as column chromatography, HPLC, recrystallization and the like.

The reaction mixture after completion of the reaction is extracted with a suitable organic solvent (e.g., ethyl acetate), washed with water and dried by a conventional method to give a crude reaction product, which is analyzed by HPLC. As a result, a peak of aldehyde (II), wherein the configuration of the asymmetric carbon, to which R₁ and R₂ are bonded, is inverted unlike the configuration of alcohol (I), can be scarcely found. Thus, the completion of the reaction without forming a stereoisomer, or completion of the reaction while markedly suppressing the formation of a stereoisomer, can be confirmed.

When the group expressed by A does not have an asymmetric carbon, the completion of the reaction without forming an optical isomer, or completion of the reaction while markedly suppressing the formation of an optical isomer, can be confirmed by the analysis based on HPLC using an optically active column, and the like, as in the above-mentioned case. Purification by HPLC using an optically active column, and the like, where necessary, for separation of optical isomer can give a peptidylaldehyde derivative having the objective configuration as a pure substance.

According to the production method of the present invention, the proportion of the objective peptidylaldehyde derivative free of inversion of configuration, unlike the peptidyl alcohol derivative of the formula (I), can be increased to not less than 95%, preferably not less than 97%, more preferably not less than 99%, of the entire amount of aldehyde produced.

The peptidyl alcohol derivative of the formula (I) can be produced by a conventional method used in the field of organic synthesis chemistry, particularly peptide synthesis chemistry. Specifically, for example, the method described in JP-A-10-147564 or an analogous method can be used for the production.

### Examples

The present invention is explained in detail in the following by referring to Examples and Reference Examples. The present invention is not limited by these examples.

### Example 1

### N-benzyloxycarbonyl-L-valyl-L-phenylalaninal

N-Benzyloxycarbonyl-L-valyl-L-phenylalaninol (9.9 g) was dissolved in DMSO (100 ml) and dichloromethane (60 ml), and diisopropylethylamine (13.3 g) was added. To this solution was added a DMSO solution (63 ml) of a sulfur trioxide-pyridine complex (16.4 g) with stirring under ice-cooling. The reaction mixture was allowed to warm to room temperature and stirred for 30 min.

After the completion of the reaction, ethyl acetate (400 ml) was added, and the reaction mixture was washed with dilute hydrochloric acid, aqueous solution of saturated sodium hydrogen carbonate and saturated brine, and dried over anhydrous magnesium sulfate. The solvent was distilled away under reduced pressure to give a white solid. The solid was washed with a mixture of hexane and ethyl acetate to give N-benzyloxycarbonyl-L-valyl-L-phenylalaninal (6.2 g, yield 62.4%) as white crystals.
HPLC purity (100.0%)
¹H-NMR (DMSO-d₆, 300 MHz) δ: 0.79 (6H, dd, J = 6.7, 2.6 Hz), 1.81-1.95 (1H, m), 2.77 (1H, dd, J = 14.5, 9.3 Hz), 3.14 (1H, dd, J = 14.0, 4.6 Hz), 3.28 (1H, S), 3.76 (1H, dd, J = 9.2, 6.6 Hz), 4.28-4.35 (1H, m), 5.02 (2H, d, J = 1.5 Hz), 7.15-7.41 (11H, m), 8.38 (1H, d, J = 7.2 Hz), 9.44 (1H, s).
MP: 132.5 - 133.6°C

| | | | | |
|---|---|---|---|---|
| Anal. (C₂₂H₂₆N₂O₄) | Calculated | C:69.09%, | H:6.85%, | N:7.32% |
| | Found | C:68.86%, | H:6.86%, | N:7.20% |

### Example 2

### N-fluorobenzenesulfonyl-L-valyl-L-leucinal

N-Fluorobenzenesulfonyl-L-valyl-L-leucinol (5.0 g) was dissolved in DMSO (50 ml) and dichloromethane (30 ml), and diisopropylethylamine (6.9 g) was added. To this solution was added a DMSO solution (36 ml) of a sulfur trioxide-pyridine complex (8.5 g) with stirring under ice-cooling. The reaction mixture was allowed to warm to room temperature and stirred for 30 min. After the completion of the reaction, ethyl acetate (200 ml) was added, and the reaction mixture was washed with dilute hydrochloric acid, aqueous solution of saturated sodium hydrogen carbonate and saturated brine, and dried over anhydrous magnesium sulfate. Ethyl acetate was distilled away under reduced pressure to give a white solid. The solid was recrystallized from ethyl acetate to give N-fluorobenzenesulfonyl-L-valyl-L-leucinal (2.84 g, yield 57.1%) as white crystals.
HPLC purity (99.8%)
¹H-NMR (DMSO-d₆, 300 MHz) b: 0.71 (3H, d, J = 6.1 Hz), 0.79-0.86 (9H, m), 1.14-1.41 (3H, m), 1.81-1.97 (1H, m), 3.58 (1H, d, J = 6.5 Hz), 3.78-3.85 (1H, m), 7.33-7.38 (2H, m), 7.78-7.83 (2H, m), 7.94 (1H, d, J = 9.5 Hz), 8.25 (1H, d, J = 7.0 Hz), 9.12 (1H, s).
MP: 156.5 - 157.3°C.

| | | | | |
|---|---|---|---|---|
| Anal. (C₁₇H₂₅FN₂O₄S) | Calculated | C:54.82%, | H:6.76%, | N:7.52% |
| | Found | C:54.67%, | H:6.78%, | N:7.51% |

### Reference Example 1

### N-benzyloxycarbonyl-L-valyl-L-phenylalaninal

N-Benzyloxycarbonyl-L-valyl-L-phenylalaninol (1.0 g) was dissolved in DMSO (6 ml) and dichloromethane (10 ml), and triethylamine (1.6 g) was added. To this solution was added a DMSO solution (6 ml) of a sulfur trioxide-pyridine complex (2.4 g) with stirring under ice-cooling. The reaction mixture was allowed to warm to room temperature and stirred for 30 min. After the completion of the reaction, ethyl acetate (100 ml) was added, and the reaction mixture was washed with dilute hydrochloric acid, aqueous solution of saturated sodium hydrogen carbonate and saturated brine, and dried over anhydrous magnesium sulfate. The solvent was distilled away under reduced pressure to give a white solid. The solid was washed with a mixture of hexane and ethyl acetate to give N-benzyloxycarbonyl-L-valyl-L-phenylalaninal (0.25 g, yield 25%) as white crystals.

HPLC purity (86%)

### Reference Example 2

### Comparative test of N-fluorobenzenesulfonyl-L-valyl-L-leucinal for stereochemical stability

### (Test method)

The following reaction mixtures 1 and 2 were stirred at room temperature, during which the reaction mixtures were sampled with the lapse of time, and N-fluorobenzenesulfonyl-L-valyl-L-leucinal and N-fluorobenzenesulfonyl-L-valyl-D-leucinal were measured by HPLC.

Reaction mixture 1: N-Fluorobenzenesulfonyl-L-valyl-L-leucinal (0.10 g) was dissolved in DMSO (1 ml) and dichloromethane (1 ml), and diisopropylethylamine (0.64 g) was added.

Reaction mixture 2: N-Fluorobenzenesulfonyl-L-valyl-L-leucinal (0.10 g) was dissolved in DMSO (1 ml) and dichloromethane (1 ml), and triethylamine (0.5 g) was added.

### HPLC conditions:

### HPLC system (LC-7A, Shimadzu Corporation)

column: YMC Pack ODS-A packed column (column length 250 mm, inner diameter 4.6 mm, manufactured by YMC).
Mobile phase: acetonitrile:purified water:trifluoroacetate (40: 60: 0.1)
Pump flow amount: 1.0 mL min⁻¹.
Detector: UV detector (SPD-10A, Shimadzu Corporation, measurement wavelength 250 nm).

### (Test result)

The ratio of N-fluorobenzenesulfonyl-L-valyl-L-leucinal to N-fluorobenzenesulfonyl-L-valyl-D-leucinal in the reaction mixture is shown in Table 1, wherein the values were determined based on the area percentage by HPLC analysis. In the co-presence of diisopropylethylamine, N-fluorobenzenesulfonyl-L-valyl-D-leucinal was found to have been hardly formed and the above compound was stereochemically stable. In the presence of triethylamine, however, N-fluorobenzenesulfonyl-L-valyl-L-leucinal was converted to N-fluorobenzenesulfonyl-L-valyl-D-leucinal by epimerization.

**Table 1**

| | Ratio of L compound* to D compound** | | | |
|---|---|---|---|---|
| | start | 30 min | 1 hr | 2 hr |
| Reaction mixture 1 | 100:0 | 99:1 | 99:1 | 99:1 |
| Reaction mixture 2 | 100:0 | 73:27 | 67:33 | 64:36 |

| | | | | |
|---|---|---|---|---|
| *: N-fluorobenzenesulfonyl-L-valyl-L-leucinal | | | | |
| **: N-fluorobenzenesulfonyl-L-valyl-D-leucinal | | | | |

The above test shows that the peptidylaldehyde derivative was stereochemically stable in the presence of diisopropylethylamine. Therefore, it is clear that, when a peptidylaldehyde derivative is to be produced, the objective peptidylaldehyde derivative can be produced without forming a stereoisomer or while suppressing the formation of a stereoisomer, by oxidizing the peptidyl alcohol derivative with activated DMSO in the presence of diisopropylethylamine.

### Industrial Applicability

According to the production method of the present invention, the formation of a stereoisomer of peptidylaldehyde derivative in the reaction mixture can be extremely suppressed or prevented during the oxidation of the compound of the formula (I), and the objective compound having a high purity can be produced in a stereochemically high yield.

This application is based on a patent application No. 10-302807 filed in Japan, the contents of which are hereby incorporated by reference.

## Claims

1. A method of producing a peptidyl aldehyde derivative of the formula (II) 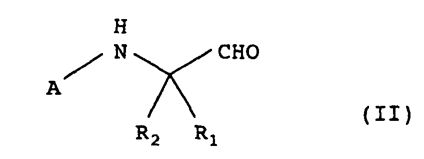 wherein A is acyl derived from amino acid, and R₁ and R₂ are different and one is hydrogen atom and the other is optionally substituted lower alkyl, which comprises oxidizing a peptidyl alcohol derivative of the formula (I) 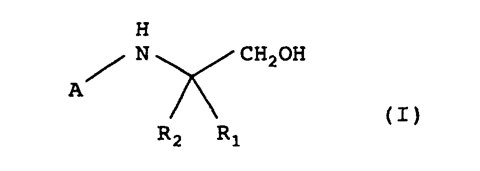 wherein A is acyl derived from amino acid, and R1 and R₂ are different and one is hydrogen atom and the other is optionally substituted lower alkyl, with activated dimethyl sulfoxide in the presence of diisopropylethylamine.
